# EUROPÄISCHE PATENTSCHRIFT

(11) **EP 3 874 311 B1**
(45) Veröffentlichungstag und Bekanntmachung des Hinweises auf die Patenterteilung: **23.09.2026**
(21) Anmeldenummer: 19790487.3
(22) Anmeldetag: 16.10.2019
(51) Int. Cl.: G02B 21/00, G02B 21/24, G02B 21/16, A61B 5/00, G03B 15/03, G03B 17/02

(54) **BILDERFASSUNGSEINRICHTUNG, SYSTEM UND VERFAHREN ZUR BILDERFASSUNG**
IMAGE-CAPTURING DEVICE, SYSTEM AND METHOD FOR CAPTURING IMAGES
DISPOSITIF D'ACQUISITION D'IMAGES, SYSTÈME ET PROCÉDÉ D'ACQUISITION D'IMAGES

(30) Priorität: 01.11.2018 DE 102018127339
(43) Veröffentlichungstag der Anmeldung: 08.09.2021
(73) Patentinhaber: Leibniz-Institut für Neurobiologie, 39118 Magdeburg (DE)
(72) Erfinder: LIPPERT, Michael, 39104 Magdeburg (DE); DECKERT, Martin, 39104 Magdeburg (DE); OHL, Frank W., 39171 Osterweddingen (DE); SCHMIDT, Bertram, 78052 Villingen-Schwenningen (DE)
(74) Vertreter: Kröncke, Rolf
(86) Internationale Anmeldenummer: PCT/EP2019/078095
(87) Internationale Veröffentlichungsnummer: WO 2020/088937

(56) Entgegenhaltungen:
- WO-A1-2014/071390
- WO-A1-2017/079688
- WO-A2-2012/027586
- JP-A- 2013 036 991
- GHOSH KUNAL K ET AL: "Miniaturized integration of a fluorescence microscope", NATURE METHODS, vol. 8, no. 10, 11 September 2011 (2011-09-11), New York, pages 871 - 878, XP055894153, ISSN: 1548-7091, Retrieved from the Internet <URL:http://www.nature.com/articles/nmeth.1694> DOI: 10.1038/nmeth.1694
- MURARI K ET AL: "Design and characterization of a miniaturized epi-illuminated microscope", PROCEEDINGS OF THE 31ST ANNUAL INTERNATIONAL CONFERENCE OF THE IEEE ENGINEERING IN MEDICINE AND BIOLOGY SOCIETY: ENGINEERING THE FUTURE OF BIOMEDICINE, EMBC 2009, IEEE, 3 September 2009 (2009-09-03), pages 5369 - 5372, XP031882437, ISBN: 978-1-4244-3296-7, DOI: 10.1109/IEMBS.2009.5334595
- ZACHARY J. SMITH ET AL: "Cell-Phone-Based Platform for Biomedical Device Development and Education Applications", PLOS ONE, vol. 6, no. 3, 2 March 2011 (2011-03-02), pages e17150, XP055203456, DOI: 10.1371/journal.pone.0017150
- BENJAMIN A FLUSBERG ET AL: "High-speed, miniaturized fluorescence microscopy in freely moving mice", NATURE METHODS, vol. 5, no. 11, 5 October 2008 (2008-10-05), pages 935 - 938, XP055121078, ISSN: 1548-7091, DOI: 10.1038/nmeth.1256

## Beschreibung

Die Erfindung betrifft eine Bilderfassungseinrichtung für die miniaturisierte Nahfeld-Bilderfassung an lebendem biologischem Gewebe, insbesondere zur Bildgebung genetischer Indikatoren, wobei die Bilderfassungseinrichtung wenigstens einen digitalen Bildsensor und eine mit dem digitalen Bildsensor zur Bilderfassung gekoppelte Objektivlinse in Form einer stabförmigen Gradientenindexlinse (GRIN) aufweist. Die Erfindung betrifft außerdem ein System für die miniaturisierte Nahfeld-Bilderfassung an biologischem Gewebe, die eine derartige Bilderfassungseinrichtung und eine mit der Bilderfassungseinrichtung verbundene Auswerteeinrichtung aufweist, sowie ein Verfahren für die miniaturisierte Nahfeld-Bilderfassung an biologischem Gewebe.

Die Erfindung betrifft allgemein das Gebiet der Bilderfassung an lebendem biologischem Gewebe, z. B. am Tier oder am Menschen. Solche Bilderfassungseinrichtungen, die auch als Fluoreszenzmikroskop oder Fluoreszenzendoskop bezeichnet werden, werden beispielsweise zur Bildgebung genetischer Aktivitätsmarker in sich frei bewegenden Mäusen und anderen Nagetieren eingesetzt. Im Gegensatz zur Verwendung von Elektroden ist durch die genetische Spezifität der Marker die Bildgebung einzeln definierter Zellpopulationen möglich. Diese Technik lässt sich daher als Komplement zur klassischen Optogenetik beschreiben: Die Zellen werden nicht oder nicht nur optisch stimuliert, sondern auch optisch ausgelesen. Werden beide Techniken miteinander kombiniert, betrifft dies das Forschungsfeld der sogenannten "Circuit Neuroscience".

Eine gattungsgemäße Bilderfassungseinrichtung ist beispielsweise aus der US 2018/0217364 A1 bekannt. Die dortige Einrichtung ist relativ großbauend, was die Einsatzmöglichkeiten einschränkt. Zudem ist ein mehrteiliger Aufbau mit zumindest einer Basisplatte vorhanden, was eine bestimmte Handhabung der Einrichtung erfordert. Aus der JP 2013 036991 A ist eine optische Erfassungseinrichtung zur Erfassung von Aktivitäten des Gehirns bekannt. Weitere Bilderfassungseinrichtungen sind bekannt aus MURARI K ET AL "Design and characterization of a miniaturized epi-illuminated microscope", PROCEEDINGS OF THE 31ST ANNUAL INTERNATIONAL CONFERENCE OF THE IEEE ENGINEERING IN MEDICINE AND BIOL-OGY SOCIETY: ENGINEERING THE FUTURE OF BIOMEDICINE, EMBC 2009, IEEE, 03. September 2009 (2009-09-03), Seiten 5369-5372, XP031882437, DOI: 10. 1109/IEMBS.2009.5334595 ISBN: 978-1-4244-3296-7, ZACHARY J. SMITH ET AL: "Cell-Phone-Based Platform for Biomedical Device Development and Education Applications", PLOS ONE, Bd.6, Nr. 3, 2. März 2011 (2011-03-02), Seite e17150, XP055203456, DOI: 10. 1371/journal.pone.0017150, BENJAMIN A FLUSBERG ET AL: "High-speed, miniaturized fluorescence microscopy in freely moving mice", NATURE METHODS, Bd. 5, Nr. 11, 5. Oktober 2008 (2008-10-05), Seiten 935-938, XP055121078, ISSN: 1548-7091, DOI: 10. 1038/nmeth. 1256. Aus der WO 2017/079688 A1 sind Systeme und Verfahren für die optogenetische Bilderfassung bekannt. Aus der WO 2012/027586 A2 ist eine Mikroskop-Bilderfassungseinrichtung mit erweiterten Bildaufnahme-Eigenschaften bekannt. Ein weiteres miniaturisiertes Fluoreszenzmikroskop ist bekannt aus der Veröffentlichung von Ghosh Kunal K et al: "Miniaturized integration of a fluorescence miscroscope", Nature Methods, Bd. 8, Nr. 10, 11. September 20211, Seiten 871-878, XP055894153, New York.

Der Erfindung liegt die Aufgabe zugrunde, eine demgegenüber verbesserte Bilderfassungseinrichtung, ein System sowie ein Verfahren zur Bilderfassung anzugeben.

Diese Aufgabe wird mit einer Bilderfassungseinrichtung gemäß Anspruch 1 gelöst. Dies beinhaltet, dass die Objektivlinse mit dem digitalen Bildsensor zu einer monolithischen, festen Baueinheit verbunden ist, wobei zwischen der Objektivlinse und dem digitalen Bildsensor keine mechanische Trennschnittstelle vorhanden ist, über die vom Anwender der digitale Bildsensor von der Objektivlinse trennbar ist. Eine solche Bilderfassungseinrichtung bietet eine Vielzahl von Vorteilen. So ist eine solche Bilderfassungseinrichtung besser miniaturisierbar, weil durch den monolithischen Aufbau in Form einer festen Baueinheit gerade besonders kleine Einzelkomponenten gut miteinander kombiniert und verbunden werden können. Ein weiterer Vorteil der Erfindung besteht darin, dass die monolithische, feste Baueinheit keine mechanische Trennschnittstelle zwischen der Objektivlinse und dem digitalen Bildsensor aufweist, sodass zwischen diesen Bauelementen immer eine feste Verbindung vorhanden ist. Dementsprechend treten keine Fokussierungsprobleme auf. Wenn z. B. wie im Stand der Technik die Objektivlinse und der digitale Bildsensor erneut zusammengesetzt werden, muss dort die Bilderfassungseinrichtung manuell nachfokussiert werden. Dies entfällt bei der erfindungsgemäßen Bilderfassungseinrichtung.

Zudem ist die erfindungsgemäße monolithische, feste Baueinheit auf diese Weise vollkommen geschützt gegen Staubeinfall, d. h. im Stand der Technik auftretende Verschmutzungseffekte der Optik können vermieden werden. Die feste Verbindung der Optik verbessert zudem die Bildstabilität in sich bewegenden Tieren und vermeidet sonstige Fehler in der optischen Verbindung.

Zudem kann eine aus solchen Bauelementen gebildete Baueinheit wesentlich kostengünstiger bereitgestellt werden, sodass sie auch als Einwegprodukt, d. h. als Wegwerfartikel, ausgebildet sein kann. Durch die besonders stark miniaturisierte Bauweise der Bilderfassungseinrichtung kann zudem das Gewicht gegenüber bekannten Einrichtungen deutlich reduziert werden. Insbesondere ist eine Volumen- und Gewichtsreduktion um mehr als den Faktor 300 möglich.

Die Bilderfassungseinrichtung kann auch ganz ohne mechanische Trennschnittstelle der zur Bilderfassungseinrichtung gehörenden Elemente ausgebildet sein, d. h. sämtliche Elemente der Bilderfassungseinrichtung sind Bestandteil der unteilbaren monolithischen festen Baueinheit.

Die Bilderfassungseinrichtung kann ohne eigenes Gehäuse ausgebildet sein. Dementsprechend sind nur die zur Bildung der Bilderfassungseinrichtung erforderlichen Komponenten, d. h. zumindest die Objektivlinse und der digitale Bildsensor, über deren Gehäuse oder sonstige mechanische Elemente miteinander verbunden. Die Bilderfassungseinrichtung kann insbesondere ohne eine gesonderte Stützstruktur zur Stabilisierung der einzelnen Komponenten ausgebildet sein.

Gemäß einer vorteilhaften Ausgestaltung der Erfindung ist vorgesehen, dass die Bilderfassungseinrichtung als einzige vom Anwender bedienbare Verbindungs- und Trennschnittstelle der Bilderfassungseinrichtung einen elektrischen Steckverbinder aufweist, mit dem die Bilderfassungseinrichtung über eine Leitung mit einer externen Stromversorgungs- und/oder Auswertungseinheit verbindbar ist. Der elektrische Steckverbinder kann direkt oder über ein kurzes Kabel mit dem digitalen Bildsensor verbunden sein. Auf diese Weise wird die Bedienbarkeit für den Anwender vereinfacht und Fehlbedienungen weitgehend vermieden. Zudem weist die Bilderfassungseinrichtung damit keine oder nur sehr wenig lose Elemente auf. Der digitale Bildsensor kann mechanisch unmittelbar mit dem elektrischen Steckverbinder verbunden sein.

Gemäß der Erfindung ist vorgesehen, dass der digitale Bildsensor als wafer-level-packaged-Bildsensor ausgebildet ist. Dies erlaubt eine extrem starke Miniaturisierung der Bilderfassungseinrichtung. Ein solcher Bildsensor ist mit Abmessungen im Bereich eines Millimeters realisierbar. Ein solcher Bildsensor ist mit integrierter Optik, d.h. mit eigener Linse, direkt auf einem Siliziumwafer hergestellt. Auf diese Weise kann ein solcher Bildsensor die Größen- und Preisgrenzen normaler Optoelektronik bei weitem unterschreiten. Beispielsweise kann der Bildsensor eine Größe von ca. 1,5 x 1,5 x 2 mm aufweisen.

Gemäß der Erfindung ist vorgesehen, dass zwischen der Objektivlinse und dem digitalen Bildsensor keine manuelle Fokussierung durch einen Anwender möglich ist. Auf diese Weise wird die herstellungsseitig vorgenommene Fokussierung permanent beibehalten. Fehleinstellungen durch den Anwender können vermieden werden. Auf diese Weise kann die Qualität der Bilderfassung deutlich verbessert werden.

Gemäß einer vorteilhaften Ausgestaltung der Erfindung ist vorgesehen, dass zwischen der Objektivlinse und dem digitalen Bildsensor ein Abstandshalter (Spacer) angeordnet ist, der in den monolithischen Aufbau der Bilderfassungseinrichtung baulich integriert ist. Durch den Abstandshalter kann der Abstand zwischen dem zu erfassenden biologischen Gewebe und den elektrischen Komponenten der Bilderfassungseinrichtung vergrößert und auf ein gewünschtes Maß gebracht werden. Dies erlaubt es, trotz der Miniaturisierung der Bilderfassungseinrichtung die elektrischen Komponenten ausreichend weit vom biologischen Gewebe zu distanzieren und unerwünschte Wechseleffekte zu vermeiden. Durch den Abstandshalter kann außerdem die Fokussierung zwischen der Objektivlinse und dem digitalen Bildsensor bereitgestellt werden.

Gemäß der Erfindung ist vorgesehen, dass zwischen der Objektivlinse und dem digitalen Bildsensor ein Strahlteiler angeordnet ist, der in den monolithischen Aufbau der Bilderfassungseinrichtung baulich integriert ist. Durch einen solchen Strahlteiler können verschiedene Lichtpfade geschaffen werden, durch die Zusatzfunktionen an der Bilderfassungseinrichtung, wie z. B. eine integrierte Beleuchtung, realisiert werden können. Der Strahlteiler ist als chroischer Strahlteiler ausgebildet, d. h. als Strahlteiler, der zwischen bestimmten Lichtfarben bzw. Wellenlängen des Lichts differenziert. Der Strahlteiler ist als dichroischer oder multichroischer Strahlteiler ausgebildet. Der Abstandshalter kann dann z. B. zwischen der Objektivlinse und dem Strahlteiler angeordnet sein.

Gemäß einer vorteilhaften Ausgestaltung der Erfindung ist vorgesehen, dass die Bilderfassungseinrichtung eine Lichtquelle in Form einer Fluoreszenzlichtquelle aufweist, die zur Lichtabgabe an den Strahlteiler eingerichtet ist, wobei die Lichtquelle in dem monolithischen Aufbau der Bilderfassungseinrichtung baulich integriert ist. Die Lichtquelle kann für verschiedene Zwecke verwendet werden, beispielsweise für die Beleuchtung des zu erfassenden Bereichs. Vorteilhafterweise kann die Lichtquelle insbesondere für die optische Stimulation genetisch manipulierter Zellen eingesetzt werden. Durch den Strahlteiler kann das Licht der Lichtquelle in den Lichtpfad eingekoppelt werden, der von der Objektivlinse zum Bildsensor führt. Die Lichtquelle kann an einer Rückenplatte montiert sein. Die Rückenplatte dient zur Ausrichtung der Lichtquelle sowie zur Kühlung.

Dadurch, dass die genannten Komponenten, insbesondere der Abstandshalter, der Strahlteiler und/oder die Lichtquelle in den monolithischen Aufbau der Bilderfassungseinrichtung baulich integriert sind, sind sie somit Teil der einstückigen festen Baueinheit. Die genannten Komponenten können damit auch zur mechanischen Stabilisierung und Abstützung des gesamten Aufbaus der Bilderfassungseinrichtung beitragen. Der digitale Bildsensor kann direkt mit einem Optikmodul der Bilderfassungseinrichtung gekoppelt sein, das den Strahlteiler, die Lichtquelle, gegebenenfalls mit einer Rückenplatte, und die Objektivlinse aufweist. Je nach Bedarf kann zusätzlich der Abstandshalter Teil des Optikmoduls sein.

Zwischen der Lichtquelle und dem Strahlteiler kann ein Kollimator angeordnet sein, beispielsweise in Form einer Linse, durch die das von der Lichtquelle beispielsweise punktförmig abgestrahlte Licht in einen parallelen Strahlengang, der auf den Strahlteiler auftrifft, umgewandelt wird. Das Licht der Lichtquelle kann auf diese Weise auf das biologische Gewebe abgestrahlt werden. Entsprechendes vom biologischen Gewebe rückgestrahltes Licht wird von der Objektivlinse aufgenommen und durchquert den Strahlteiler vertikal in Richtung des digitalen Bildsensors, wo es aufgezeichnet werden kann.

Gemäß einer vorteilhaften Ausgestaltung der Erfindung ist vorgesehen, dass im Strahlengang zwischen der Objektivlinse und einer Bilderfassungsoberfläche des digitalen Bildsensors kein reelles Zwischenbild des von der Objektivlinse aufgenommenen Bilds erzeugt ist. Die ist Objektivlinse dazu eingerichtet, das von der Objektivlinse aufgenommene Bild in Richtung des digitalen Bildsensors ins Unendliche zu fokussieren.

Gemäß einer vorteilhaften Ausgestaltung der Erfindung ist vorgesehen, dass die zu einer monolithischen, festen Baueinheit miteinander verbundenen Komponenten der Bilderfassungseinrichtung mittels Adhäsiv und/oder mittels reibschlüssiger Verbindung miteinander verbunden sind. Das Adhäsiv kann ein Klebstoff sein, z. B. Sekundenkleber. Die reibschlüssige Verbindung kann beispielsweise eine Klemm-Verbindung sein.

Gemäß einer vorteilhaften Ausgestaltung der Erfindung ist vorgesehen, dass die Bilderfassungseinrichtung als Einwegprodukt ausgebildet ist. Dies hat den Vorteil, dass ein komplizierter Aufbau mit einzelnen wechselbaren Komponenten vermieden werden kann und hierdurch die Anwendung der Bilderfassungseinrichtung vereinfacht wird und zuverlässiger gestaltet werden kann.

Streulicht im Strahlengang der Bilderfassungseinrichtung kann beispielsweise durch Blenden unterdrückt werden. Eine Blende kann beispielsweise auch im Abstandshalter enthalten sein. Streulicht kann zusätzlich durch eine absorbierende Beschichtung unterdrückt werden, bzw. durch im Klebstoff enthaltene Pigmente reduziert werden.

Der Strahlengang in der erfindungsgemäßen Bilderfassungseinrichtung kann beispielsweise einem Epifluoreszenzmikroskop entsprechen, wobei bei der erfindungsgemäßen Bilderfassungseinrichtung kein reelles Zwischenbild zwischen der Objektivlinse und dem digitalen Bildsensor erzeugt wird. Das von der Lichtquelle zugeführte Anregungslicht kann in eine leicht zur Bildgebungsebene versetzte Ebene projiziert werden. Das vom biologischen Gewebe wieder abgegebene Emissionslicht wird von der Objektivlinse etwa ins Unendliche fokussiert und durch eine Linse des digitalen Bildsensors auf dessen Bilderfassungsoberfläche abgebildet.

Der digitale Bildsensor kann wie eine Miniaturkamera ausgebildet sein. Zu diesem Zweck kann der digitale Bildsensor außer dem eigentlichen Sensorelement noch eine integrierte Linse aufweisen.

Das Anregungslicht der Lichtquelle kann im Optikmodul bandpassgefiltert werden, das von dem biologischen Gewebe abgegebene Emissionslicht kann ebenfalls entweder bandpassgefiltert oder langpassgefiltert werden.

Der Strahlteiler kann mit einer Trennwellenlänge ausgebildet sein, die etwa mittig zwischen dem Anregungslicht und dem Emissionslicht ist. Im Strahlengang können sich Streulichtblenden befinden, z. B. am Übergang von der Lichtquelle zum Strahlteiler, beispielsweise am Tubus, und/oder direkt vor dem digitalen Bildsensor und/oder vor der Objektivlinse.

Die Bilderfassungseinrichtung kann je nach Ausführungsform mit eigener elektrischer Energiequelle und/oder einem Speicher für die erfassten Bilder realisiert werden. Alternativ oder zusätzlich können Daten und/oder elektrische Energie über ein Kabel übertragen werden, über das die Bilderfassungseinrichtung mit einer Auswerteeinrichtung verbunden ist. Das Kabel kann durch einen elektrischen Stecker mit dem Steckverbinder der Bilderfassungseinrichtung verbunden werden. Das Kabel kann über eine Drehentlastung, z. B. einem Schleifring, verfügen und ist mit der Auswerteeinrichtung verbunden. Die Auswerteeinrichtung kann zur Aufnahme der übertragenen Daten und zur Anzeige der Daten ausgebildet sein.

Die Verwendung eines wafer-level-packaged-Bildsensors führt zu enormen Größen- und Gewichtsvorteilen. So kann mindestens der eigentliche Sensor sowie der Schaltkreis, der den Sensor steuert, direkt auf Chipebene miteinander verbunden sein und eine Einheit bilden. Auf diese Weise sind nur wenige Leitungen zur Datenübertragung nötig. Der digitale Bildsensor kann als Kamera ausgebildet sein und in diesem Fall direkt eine Linse enthalten. Es kann auch ein zusätzliches beugendes Element (Linse, Blende, defraktives Element) in der Bilderfassungseinrichtung zwischen dem Bildsensor und dem Optikmodul, insbesondere der Objektivlinse, verbaut sein. Es kann bereits ein Mikrocomputersystem in der Bilderfassungseinrichtung integriert sein, das die erfassten Daten des Bildsensors automatisch analysiert, z. B. im Hinblick auf Zellextraktion oder Detektion von Helligkeitstransienten. Auf diese Weise kann die notwendige Bandbreite für die Datenübertragung von der Bilderfassungseinrichtung zur Auswerteeinrichtung reduziert werden.

Die Bilderfassungseinrichtung kann in einer Form realisiert werden, die günstig für die Befestigung auf einem Versuchstier ist, z. B. mittels Adhäsiv oder durch Reibung. Hierzu kann die äußere Oberfläche der Bilderfassungseinrichtung entweder rau oder uneben sein, oder sie kann Vorsprünge aufweisen, die ein Klebstoff umfließen kann.

Wird die Bilderfassungseinrichtung ohne eigenes Gehäuse realisiert, kann sie vor äußeren Lichteinflüssen durch Beschichtung mit einer lichtabsorbierenden Masse geschützt werden. Die lichtabsorbierende Masse kann beispielsweise auf die Außenseite der Bilderfassungseinrichtung aufgespritzt oder in anderer Weise aufgetragen werden. Auf diese Weise ist ein kompletter einhausender Körper der Bilderfassungseinrichtung nicht notwendig.

Die Lichtquelle kann eine Halbleiterlichtquelle sein, z. B. eine **LED,** eine Laserdiode oder ein VCSEL-Laser. Die Lichtquelle kann insbesondere zur zumindest annähernd parallelen Abstrahlung des Lichts eingerichtet sein, z. B. indem die Lichtquelle einen Parabolspiegel aufweist oder eine Linse. Der Kollimator kann als Linse, GRIN-Element oder diffraktives Element ausgebildet sein, oder als Kombination aus solchen Elementen.

Die Lichtquelle kann zur Versorgung mit elektrischer Energie ebenfalls mit dem elektrischen Steckverbinder der Bilderfassungseinrichtung verbunden sein. Diese elektrische Verbindung kann beispielsweise durch ein dünnes Kabel realisiert sein, z. B. ein Polyimid-Flexkabel oder in Form einer elektrisch leitfähigen Beschichtung. Die elektrisch leitfähige Beschichtung kann z. B. durch einen räumlichen Druckvorgang realisiert sein, z. B. Aerosol-Direktschreiben. Es kann auch eine Metallisierung auf der Außenoberfläche aufgedampft sein.

Die verwendeten Anregungs- und Emissionsfilter können Interferenzfilter und/oder absorbierende Farbfilter sein. Diese Filter können direkt auf optischen Oberflächen der Bilderfassungseinrichtung aufgebracht sein oder im Körper von optischen Elementen, z. B. Linsen als Farbstoff gelöst sein.

Der Strahlteiler ist aus Glas oder Kunststoff gebildet, z. B. aus einem Polymer. Er kann beispielsweise eine Würfelform oder eine Quaderform aufweisen. Die die optische Filterung bewirkende Schicht kann beispielsweise aufgedampft oder aufgespritzt sein. Die Filterschicht kann auch als dünne Folie realisiert sein, z. B. als Folie mit einer Schichtdicke von weniger als 200 µm.

Die Teile der Bilderfassungseinrichtung, insbesondere die optisch wirksamen Bauelemente, können durch Kunststoff-Spritzgussverfahren und/oder durch 3D-Druckverfahren hergestellt werden.

Die Objektivlinse kann die Objektebene auch direkt in die Ebene der Bilderfassungsoberfläche des digitalen Bildsensors abbilden. Die Objektivlinse hat eine Erfassungsseite, an der das Licht der Lichtquelle abgestrahlt werden kann und auf dieser Seite empfangenes Licht dem digitalen Bildsensor zugeführt werden kann. Die Objektivlinse kann an der Erfassungsseite eine Lichtablenkvorrichtung aufweisen, z. B. ein Mikroprisma oder ein Gitter, befestigt ist, welches die Bildgebung zur Seite hin erlaubt, d. h. in einem bestimmten Winkel zur Längsachse der Objektivlinse.

Die Auswerteeinrichtung kann eine Software zur Analyse der Daten aufweisen.

Die Erfindung betrifft außerdem ein System für die miniaturisierte Nahfeld-Bilderfassung an biologischem Gewebe, die eine Bilderfassungseinrichtung der zuvor erläuterten Art und eine mit der Bilderfassungseinrichtung verbundene Auswerteeinrichtung aufweist. Auch hierdurch können die zuvor erläuterten Vorteile realisiert werden.

Die Erfindung betrifft außerdem ein Verfahren zur miniaturisierten Nahfeld-Bilderfassung an biologischem Gewebe, insbesondere für die Bildgebung genetischer Indikatoren in dem biologischen Gewebe, wobei die Bilderfassung mittels einer Bilderfassungseinrichtung der zuvor erläuterten Art durchgeführt wird. Auch hierdurch können die zuvor erläuterten Vorteile realisiert werden.

Die Erfindung kann genetische Aktivitätsmarker verwenden, die im Gehirn von Säugetieren oder Menschen detektiert werden können, um entsprechende Rückschlüsse auf die Prozesse innerhalb der sie exprimierenden Zellen zu ziehen. Beispielsweise lässt sich die Erhöhung der Fluoreszenz von genetischen Calciumindikatoren messen, die bei neuronaler Aktivität in Nervenzellen durch freiwerdendes Calcium auftritt, und damit die neuronale Aktivität detektieren. Hierzu nimmt die Bilderfassungseinrichtung fortlaufend Bilder des direkt unter der Objektivlinse liegenden und durch die integrierte Lichtquelle beleuchteten Gewebes auf.

Die Aktivitätsmarker oder die entsprechende Erbinformation sind entweder bereits im Tier enthalten - wenn es sich um eine entsprechende transgene Tierlinie handelt - oder werden durch eine gentechnische Methode (z. B. viralen Vektor, Elektroporation) in die zu untersuchenden Zellen gebracht. Dazu, und insbesondere zur anschließenden Implantation, wird der Schädel eröffnet. Die Objektivlinse wird dann so im Gewebe implantiert, dass sie den transgenen Bereich abdeckt, also die Marker in den Zellen von Interesse abbilden kann. Danach wird das noch freiliegende Gehirn durch eine Schutzschicht aus z. B. Silikon geschützt und der noch exponierte Teil des Implantats - durch z. B. einen Zement-Fix mit dem Schädel bzw. einer, relativ zum untersuchten Gewebe, rigiden Struktur verbunden, um ein Verwackeln der Bilder zu vermeiden. Anschließend erfolgt unter Umständen noch ein weitergehender Wundverschluss. Im Fall, dass die Zellen den genetischen Marker nach der Implantation erst herstellen müssen, schließt sich eine Warteperiode an, bis der Marker in ausreichender Konzentration vorliegt. Typischerweise ist eine Wartezeit von ca. 2-4 Wochen allerdings ohnehin nötig, bis das Gewebe vor der Linse sich vom Eingriff erholt hat und Aktivität auf Einzelzellebene sichtbar wird.

Die Bilderfassungseinrichtung nimmt den Bereich von Interesse im Anschließenden entweder im Kontext eines bestimmten neurobiologischen Experiments oder während des normalen Verhaltens auf. Vorteilhaft sind Bildwiederholraten von 1-100 Hz, meist 5-30 Hz. Die Dauer der Aufnahme beträgt typischerweise Minuten bis Stunden, je nach Fragestellung kann die Aufnahme aber auch durchgängig sein - beispielsweise als Gehirn-Maschinen-Schnittstelle, um kontinuierlich Gehirnaktivität aus dem Gehirn von gelähmten Patienten auszulesen. Erfolgt die Aufnahme nicht kontinuierlich, wird die Bilderfassungseinrichtung am Steckverbinder getrennt, sodass das Verbindungskabel das Subjekt nicht weiter einschränkt. Für den nächsten Versuch wird das Verbindungskabel entsprechend wieder angesteckt.

Die Bilderfassungseinrichtung kann einen oder mehrere weitere Sensoren aufweisen, z. B. wenigstens einen Calciumsensor und/oder wenigstens einen Spannungssensor. Mittels solcher Sensoren kann die neuronale Aktivität in Nervenzellen erfasst werden.

Die Erfindung wird nachfolgend anhand von Ausführungsbeispielen unter Verwendung von Zeichnungen näher erläutert.

Es zeigen
- Figur 1: eine Bilderfassungseinrichtung in Seitenansicht und
- Figur 2: ein System zur Bilderfassung und
- Figur 3: die Bilderfassungseinrichtung gemäß Figur 1 mit weiteren Details.

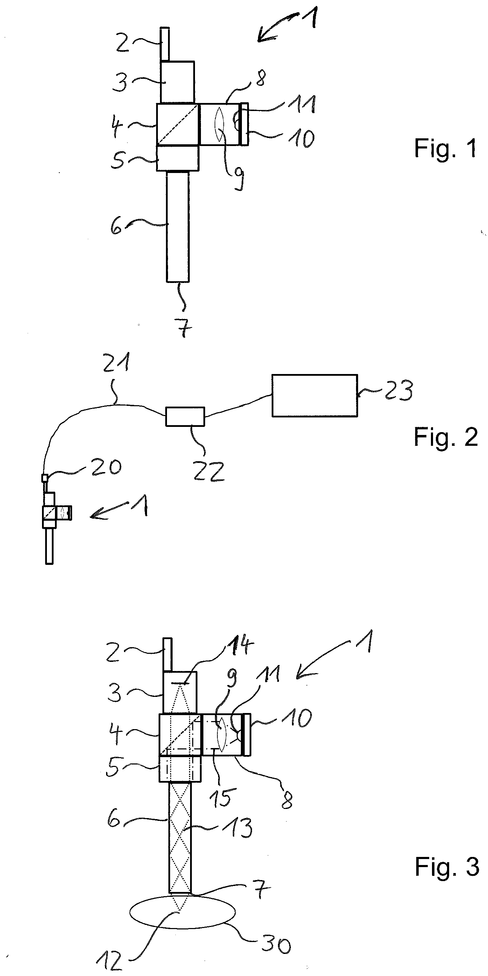

Die Bilderfassungseinrichtung 1 gemäß Figur 1 weist einen digitalen Bildsensor 3 auf, der als wafer-level-packaged-Bildsensor ausgebildet sein kann. An dem Bildsensor 3 ist ein elektrischer Steckverbinder 2 angeordnet, z. B. in Form einer Steckbuchse. Der Bildsensor 3 ist an seiner zur Bilderfassung eingerichteten Seite mit einem Strahlteiler 4 gekoppelt. Der Strahlteiler 4 ist als chroischer Strahlteiler ausgebildet, d. h. als Strahlteiler, der zwischen bestimmten Lichtfarben bzw. Wellenlängen des Lichts differenziert.

An dem Strahlteiler 4 ist außerdem eine Lichtquelle 11 angebracht. Die Lichtquelle 11 ist an einer Rückenplatte 10 montiert. Der Anordnung aus der Rückenplatte 10 mit der Lichtquelle 11 ist über eine Linsenhalterung 8 mit dem Strahlteiler 4 verbunden. In der Linsenhalterung 8 kann eine Linse 9 angeordnet sein, z. B. eine Kollimationslinse, durch die von der Lichtquelle 11 im Wesentlichen punktförmig abgegebenes Licht in einen im Wesentlichen parallelen Strahlenverlauf gewandelt wird.

Im weiteren Strahlengang von dem Bildsensor 3 hin zu einem Bildaufnahmebereich 7 der Bilderfassungseinrichtung 1 ist eine Objektivlinse 6 angeordnet, die über einen Abstandshalter 5 mit dem Strahlteiler 4 gekoppelt ist. Die Objektivlinse 6 ist als stabförmige Gradientenindexlinse ausgebildet.

Bei dem beschriebenen Aufbau der Bilderfassungseinrichtung 1 können die Objektivlinse 6, der Abstandshalter 5 (der optional ist), die Lichtquelle 11 mit der Rückenplatte 10 und der Linsenhalterung 8 mit dem Strahlteiler 4 zu einem monolithischen Optikmodul gekoppelt sein. Dieses Optikmodul ist mit dem digitalen Bildsensor 3 zu einer monolithischen festen Baueinheit verbunden.

Die Figur 2 zeigt ein System zur Bilderfassung, das eine Bilderfassungseinrichtung 1 und eine Auswerteeinrichtung 23 aufweist. Die Auswerteeinrichtung 23 ist über ein Kabel 21 mit der Bilderfassungseinrichtung 1 gekoppelt. Hierfür weist das Kabel 21 endseitig einen Stecker 20 auf, der mit dem elektrischen Steckverbinder 2 der Bilderfassungseinrichtung 1 gekoppelt ist. Zusätzlich kann das Kabel 21 eine Drehentlastung 22 aufweisen, um Torsionsbelastungen des Kabels 21 zu minimieren. Mittels der Bilderfassungseinrichtung 1 können über den Bildaufnahmebereich 7 Bilder erfasst werden und über das Kabel 21 an die Auswerteeinrichtung 23 übertragen werden. In der Auswerteeinrichtung 23 können die erfassten Bilder ausgewertet werden.

Zudem kann die Auswerteeinrichtung 23 zur Steuerung der Bilderfassungseinrichtung, z. B. zur Ansteuerung der Lichtquelle 11, eingerichtet sein und entsprechende Steuersignale an die Bilderfassungseinrichtung 1 abgeben.

Die Figur 3 zeigt die Bilderfassungseinrichtung 1 mit darin eingezeichneten Licht-Strahlenverläufen. Bei dem digitalen Bildsensor 3 ist zusätzlich dessen Bilderfassungsoberfläche 14 eingezeichnet.

Von der Lichtquelle 11 abgegebenes Licht wird über die Linse 9 in einen im Wesentlichen parallelen Strahlenverlauf 15 umgewandelt. Der Strahlenverlauf 15 wird über den Strahlteiler 4 in Richtung der Objektivlinse 6 abgelenkt und durch die Objektivlinse 6 im Bildaufnahmebereich 7 abgestrahlt. Das abgestrahlte Licht trifft auf biologisches Gewebe 30. Von dort wiederum abgegebenes Licht wird an einer Stelle 12, auf die die Objektivlinse 6 fokussiert, aufgenommen und über die Objektivlinse 6 mittels des eingezeichneten über Kreuz verlaufenden Strahlengangs 13 weitergeleitet. Die Objektivlinse 6 fokussiert in Richtung des digitalen Bildsensors 3 ins Unendliche, d. h. es wird über den Abstandshalter 5 und den Strahlteiler 4 ein im Wesentlichen paralleler Strahlenverlauf weitergeleitet. Über eine im digitalen Bildsensor 3 integrierte Linse wird der ankommende parallele Strahlenverlauf auf die Bilderfassungsoberfläche 14 fokussiert.

## Patentansprüche

1. Bilderfassungseinrichtung (1) für die miniaturisierte Nahfeld-Bilderfassung an biologischem Gewebe, insbesondere zur Bildgebung genetischer Indikatoren,
- wobei die Bilderfassungseinrichtung (1) wenigstens einen digitalen Bildsensor (3) hat, der als wafer-level-packaged Bildsensor mit integrierter Optik mit eigener Linse direkt auf einem Siliziumwafer ausgebildet ist,
- und eine mit dem digitalen Bildsensor (3) zur Bilderfassung gekoppelte Objektivlinse (6) in Form einer stabförmigen Gradientenindexlinse (GRIN) aufweist, wobei die Objektivlinse (6) dazu eingerichtet sein, das von der Objektivlinse (6) aufgenommene Bild in Richtung des digitalen Bildsensors (3) ins Unendliche zu fokussieren, wobei über die im digitalen Bildsensor (3) integrierte Linse der ankommende parallele Strahlenverlauf auf die Bilderfassungsoberfläche (14) des digitalen Bildsensors (3) fokussiert ist,
- wobei die Objektivlinse (6) mit dem digitalen Bildsensor (3) zu einer monolithischen, festen Baueinheit verbunden ist,
- wobei zwischen der Objektivlinse (6) und dem digitalen Bildsensor (3) ein dichroischer oder multichroischer Strahlteiler (4) angeordnet ist, der der zwischen bestimmten Lichtfarben bzw. Wellenlängen des Lichts differenziert, wobei der Strahlteiler (4) in den monolithischen Aufbau der Bilderfassungseinrichtung (1) baulich integriert ist, wobei der Strahlteiler aus Glas oder Kunststoff gebildet ist,
- wobei die Bilderfassungseinrichtung (1) eine Lichtquelle (11) in Form einer Fluoreszenzlichtquelle aufweist, die zur Lichtabgabe an den Strahlteiler (4) eingerichtet ist, wobei die Lichtquelle (4) in den monolithischen Aufbau der Bilderfassungseinrichtung (1) baulich integriert ist,
- wobei durch den Strahlteiler (4) das Licht der Lichtquelle (11) in den Lichtpfad eingekoppelt wird, der von der Objektivlinse (6) zum Bildsensor (3) führt,
- wobei zwischen der Objektivlinse (6) und dem digitalen Bildsensor (3) keine mechanische Trennschnittstelle vorhanden ist, über die vom Anwender der digitale Bildsensor (3) von der Objektivlinse (6) trennbar ist,
- wobei zwischen der Objektivlinse (6) und dem digitalen Bildsensor (3) keine manuelle Fokussierung durch einen Anwender möglich ist.

2. Bilderfassungseinrichtung nach Anspruch 1, **dadurch gekennzeichnet, dass** die Bilderfassungseinrichtung (1) als einzige vom Anwender bedienbare Verbindungs- und Trennschnittstelle der Bilderfassungseinrichtung (1) einen elektrischen Steckverbinder (2) aufweist, mit dem die Bilderfassungseinrichtung (1) über eine Leitung (21) mit einer externen Stromversorgungs- und/oder Auswertungseinheit (23) verbindbar ist.

3. Bilderfassungseinrichtung nach einem der vorhergehenden Ansprüche, **dadurch gekennzeichnet, dass** zwischen der Objektivlinse (6) und dem digitalen Bildsensor (3) ein Abstandshalter (5, Spacer) angeordnet ist, der in den monolithischen Aufbau der Bilderfassungseinrichtung (1) baulich integriert ist, wobei durch den Abstandshalter (5) die Fokussierung zwischen der Objektivlinse (6) und dem digitalen Bildsensor (3) bereitgestellt ist.

4. Bilderfassungseinrichtung nach einem der vorhergehenden Ansprüche, **dadurch gekennzeichnet, dass** im Strahlengang zwischen der Objektivlinse (6) und einer Bilderfassungsoberfläche (14) des digitalen Bildsensors (3) kein reelles Zwischenbild des von der Objektivlinse (6) aufgenommenen Bilds erzeugt ist.

5. Bilderfassungseinrichtung nach einem der vorhergehenden Ansprüche, **dadurch gekennzeichnet, dass** die zu einer monolithischen, festen Baueinheit miteinander verbundenen Komponenten der Bilderfassungseinrichtung (1) mittels Adhäsiv und/oder mittels reibschlüssiger Verbindung miteinander verbunden sind.

6. Bilderfassungseinrichtung nach einem der vorhergehenden Ansprüche, **dadurch gekennzeichnet, dass** die Bilderfassungseinrichtung (1) als Einwegprodukt ausgebildet ist.

7. System für die miniaturisierte Nahfeld-Bilderfassung an biologischem Gewebe, die eine Bilderfassungseinrichtung (1) nach einem der vorhergehenden Ansprüche und eine mit der Bilderfassungseinrichtung (1) verbundene Auswerteeinrichtung (23) aufweist.

8. Verfahren zur miniaturisierten Nahfeld-Bilderfassung an biologischem Gewebe, insbesondere für die Bildgebung genetischer Indikatoren in dem biologischem Gewebe, **dadurch gekennzeichnet, dass** die Bilderfassung mittels einer Bilderfassungseinrichtung (1) nach einem der Ansprüche 1 bis 6 durchgeführt wird.

## Claims

1. An imaging device (1) for miniaturized near-field imaging on biological tissue, in particular for imaging of genetic indicators,
- wherein the imaging device (1) has at least one digital image sensor (3) which is formed as a wafer-level-packaged image sensor with integrated optics with its own lens directly on a silicon wafer,
- and an objective lens (6) coupled to the digital image sensor (3) for imaging, in the form of a rod-shaped gradient-index lens (GRIN),
- wherein the objective lens (6) is configured to focus the image captured by the objective lens (6) to infinity in the direction of the digital image sensor (3), wherein via the lens integrated in the digital image sensor (3), the incoming parallel beam path is focused onto the imaging surface (14) of the digital image sensor (3),
- wherein the objective lens (6) is connected to the digital image sensor (3) to form a monolithic, solid structural unit,
- wherein a dichroic or multichroic beam splitter (4) is arranged between the objective lens (6) and the digital image sensor (3), which differentiates between specific light colors or wavelengths of light,
- wherein the beam splitter (4) is structurally integrated into the monolithic structure of the imaging device (1), wherein the beam splitter is made of glass or plastic,
- wherein the imaging device (1) comprises a light source (11) in the form of a fluorescence light source, which is configured for light emission to the beam splitter (4), wherein the light source (11) is structurally integrated into the monolithic structure of the imaging device (1),
- wherein by means of the beam splitter (4), the light from the light source (11) is coupled into the light path that leads from the objective lens (6) to the image sensor (3),
- wherein between the objective lens (6) and the digital image sensor (3) there is no mechanical separation interface via which the digital image sensor (3) can be separated from the objective lens (6) by the user,
- wherein between the objective lens (6) and the digital image sensor (3) no manual focusing by a user is possible.

2. The imaging device according to claim 1, **characterized in that** the imaging device (1) comprises, as the only user-operable connection and disconnection interface, an electrical connector (2) with which the imaging device (1) is connectable via a line (21) to an external power supply and/or evaluation unit (23).

3. The imaging device according to one of the preceding claims, **characterized in that** a spacer (5) is arranged between the objective lens (6) and the digital image sensor (3), which is structurally integrated into the monolithic structure of the imaging device (1), wherein the focusing between the objective lens (6) and the digital image sensor (3) is provided by the spacer (5).

4. The imaging device according to one of the preceding claims, **characterized in that** no real intermediate image of the image captured by the objective lens (6) is generated in the beam path between the objective lens (6) and an imaging surface (14) of the digital image sensor (3).

5. The imaging device according to one of the preceding claims, **characterized in that** the components of the imaging device (1) connected to one another to form a monolithic, solid structural unit are connected to one another by means of adhesive and/or by means of a friction-locked connection.

6. The imaging device according to one of the preceding claims, **characterized in that** the imaging device (1) is designed as a disposable product.

7. A system for miniaturized near-field imaging on biological tissue, comprising an imaging device (1) according to one of the preceding claims and an evaluation unit (23) connected to the imaging device (1).

8. A method for miniaturized near-field imaging on biological tissue, in particular for the imaging of genetic indicators in the biological tissue, **characterized in that** the imaging is performed by means of an imaging device (1) according to one of claims 1 to 6.

## Revendications

1. Dispositif d'acquisition d'images (1) pour l'imagerie miniaturisée en champ proche sur du tissu biologique, en particulier pour l'imagerie d'indicateurs génétiques,
- dans lequel le dispositif d'acquisition d'images (1) possède au moins un capteur d'image numérique (3) qui est réalisé sous la forme d'un capteur d'image encapsulé au niveau de la tranche (« wafer-level-packaged ») avec une optique intégrée dotée de sa propre lentille, directement sur une tranche de silicium,
- et une lentille d'objectif (6) couplée au capteur d'image numérique (3) pour l'acquisition d'images, sous la forme d'une lentille à gradient d'indice (GRIN) en forme de tige,
- dans lequel la lentille d'objectif (6) est conçue pour focaliser l'image capturée par la lentille d'objectif (6) à l'infini en direction du capteur d'image numérique (3), où, par le biais de la lentille intégrée dans le capteur d'image numérique (3), le trajet de faisceau parallèle entrant est focalisé sur la surface d'acquisition d'images (14) du capteur d'image numérique (3),
- dans lequel la lentille d'objectif (6) est reliée au capteur d'image numérique (3) pour former une unité structurelle monolithique et solide,
- dans lequel un diviseur de faisceau dichroïque ou multichroïque (4) est disposé entre la lentille d'objectif (6) et le capteur d'image numérique (3), qui différencie des couleurs de lumière ou des longueurs d'onde de lumière spécifiques,
- dans lequel le diviseur de faisceau (4) est intégré structurellement dans la structure monolithique du dispositif d'acquisition d'images (1), le diviseur de faisceau étant en verre ou en matière plastique,
- dans lequel le dispositif d'acquisition d'images (1) comprend une source de lumière (11) sous la forme d'une source de lumière de fluorescence, qui est configurée pour émettre de la lumière vers le diviseur de faisceau (4), la source de lumière (11) étant intégrée structurellement dans la structure monolithique du dispositif d'acquisition d'images (1),
- dans lequel, par le biais du diviseur de faisceau (4), la lumière de la source de lumière (11) est couplée dans le chemin optique qui mène de la lentille d'objectif (6) au capteur d'image (3),
- dans lequel, entre la lentille d'objectif (6) et le capteur d'image numérique (3), il n'existe aucune interface de séparation mécanique par laquelle le capteur d'image numérique (3) peut être séparé de la lentille d'objectif (6) par l'utilisateur,
- dans lequel, entre la lentille d'objectif (6) et le capteur d'image numérique (3), aucune mise au point manuelle par un utilisateur n'est possible.

2. Dispositif d'acquisition d'images selon la revendication 1, **caractérisé en ce que** le dispositif d'acquisition d'images (1) comprend, comme seule interface de connexion et de déconnexion actionnable par l'utilisateur, un connecteur électrique (2) avec lequel le dispositif d'acquisition d'images (1) peut être connecté via une ligne (21) à une unité d'alimentation électrique et/ou d'évaluation externe (23).

3. Dispositif d'acquisition d'images selon l'une des revendications précédentes, **caractérisé en ce qu'**une entretoise (5, spacer) est disposée entre la lentille d'objectif (6) et le capteur d'image numérique (3), laquelle est intégrée structurellement dans la structure monolithique du dispositif d'acquisition d'images (1), dans lequel la mise au point entre la lentille d'objectif (6) et le capteur d'image numérique (3) est assurée par l'entretoise (5).

4. Dispositif d'acquisition d'images selon l'une des revendications précédentes, **caractérisé en ce qu'**aucune image intermédiaire réelle de l'image capturée par la lentille d'objectif (6) n'est générée dans le chemin de faisceau entre la lentille d'objectif (6) et une surface d'acquisition d'images (14) du capteur d'image numérique (3).

5. Dispositif d'acquisition d'images selon l'une des revendications précédentes, **caractérisé en ce que** les composants du dispositif d'acquisition d'images (1) reliés les uns aux autres pour former une unité structurelle monolithique et solide sont reliés les uns aux autres au moyen d'un adhésif et/ou au moyen d'une liaison par friction.

6. Dispositif d'acquisition d'images selon l'une des revendications précédentes, **caractérisé en ce que** le dispositif d'acquisition d'images (1) est conçu comme un produit à usage unique.

7. Système pour l'imagerie miniaturisée en champ proche sur du tissu biologique, comprenant un dispositif d'acquisition d'images (1) selon l'une des revendications précédentes et une unité d'évaluation (23) connectée au dispositif d'acquisition d'images (1).

8. Procédé pour l'imagerie miniaturisée en champ proche sur du tissu biologique, en particulier pour l'imagerie d'indicateurs génétiques dans le tissu biologique, **caractérisé en ce que** l'acquisition d'images est réalisée au moyen d'un dispositif d'acquisition d'images (1) selon l'une des revendications 1 à 6.
